**Europäisches Patentamt**

**European Patent Office**

**Office européen des brevets**

(11) Veröffentlichungsnummer : **0 327 850 B1**

(12) # EUROPÄISCHE PATENTSCHRIFT

(45) Veröffentlichungstag der Patentschrift :
**15.04.92 Patentblatt 92/16**

(51) Int. Cl.$^5$ : **A61M 37/00, F16L 33/00**

(21) Anmeldenummer : **89100863.3**

(22) Anmeldetag : **19.01.89**

(54) **Katheterkupplung.**

(30) Priorität : **09.02.88 DE 8801583 U**

(43) Veröffentlichungstag der Anmeldung :
**16.08.89 Patentblatt 89/33**

(45) Bekanntmachung des Hinweises auf die
Patenterteilung :
**15.04.92 Patentblatt 92/16**

(84) Benannte Vertragsstaaten :
**AT BE CH DE ES FR GB GR IT LI LU NL SE**

(56) Entgegenhaltungen :
**EP-A- 0 258 580**
**DE-A- 3 102 142**

(73) Patentinhaber : **B. Braun Melsungen AG**
**Carl-Braun Strasse**
**W-3508 Melsungen (DE)**

(72) Erfinder : **Koch, Heinrich, Dr.**
**Haarbreite 41**
**W-3436 Hess. Lichtenau (DE)**
Erfinder : **Fuchs, Jürgen**
**Wolfhager Strasse 45**
**W-3501 Emstal-Sand (DE)**

(74) Vertreter : **Selting, Günther, Dipl.-Ing. et al**
**Patentanwälte von Kreisler, Selting, Werner**
**Deichmannhaus am Hauptbahnhof**
**W-5000 Köln 1 (DE)**

EP 0 327 850 B1

## Beschreibung

Die Erfindung betrifft eine Katheterkupplung nach dem Oberbegriff des Anspruchs 1.

Eine bekannte Katheterkupplung dieser Art (DE-PS 31 02 142) weist an einem Anschlußgehäuse einen hohlen Anschlußstutzen auf, in den der Katheter eingeführt werden kann. Im Inneren des Anschlußstutzens sitzt ein elastomeres Klemmstück mit einem Kanal zum Hindurchführen des Katheters. Dieses Klemmstück wird durch ein als Überwurfmutter ausgebildetes Druckstück beim Aufschrauben dieses Druckstücks auf den Anschlußstutzen axial zusammengedrückt, wobei es sich radial verformt und den Katheter im Anschlußstutzen festklemmt. Der Kanal des Klemmstücks wird durch den axialen Druck im Durchmesser reduziert, wodurch das Klemmstück um den eingeführten Katheterschlauch herum gestaucht wird. Diese Katheterkupplung funktioniert nur sicher, wenn als Katheter harte, formstabile Schläuche verwendet werden; weichere Schläuche werden zusammengedrückt, so daß sich das Katheterlumen verkleinert ohne daß jedoch die gewünschte zugfeste Verbindung erreicht wird. Wegen der begrenzten Länge des Klemmstücks wird der Katheter nur in einem wenige Millimeter langen Bereich seines proximalen Endes gepreßt und festgehalten, was zu Undichtigkeiten der Katheterkupplung führen kann, insbesondere wenn der Katheter aus einem relativ weichem Material besteht. Ein weiterer Nachteil der bekannten Katheterkupplung liegt darin, daß das elastomere Klemmstück bei abgeschraubtem Druckstück aus dem Anschlußstutzen herausfallen kann. In der Praxis werden oft Anschlußstutzen und Klemmstück einzeln auf den Katheter aufgefädelt, um dann gemeinsam an dem Anschlußgehäuse befestigt zu werden. Dieses Einfädeln bereitet insbesondere bei kleinlumigen Kathetern und entsprechend kleinformatigen Kanälen Schwierigkeiten.

Der Erfindung liegt die Aufgabe zugrunde, eine Katheterkupplung der im Oberbegriff des Anspruchs 1 angegebenen Art zu schaffen, die leicht und ohne Schwierigkeiten gehandhabt werden kann und eine sichere Abdichtung des Katheters zum Anschlußstutzen über eine größere Länge ermöglicht.

Die Lösung dieser Aufgabe erfolgt erfindungsgemäß mit den im kennzeichnenden Teil des Anspruchs 1 angegebenen Merkmalen.

Bei der erfindungsgemäßen Katheterkupplung hat das Klemmstück, das beim Festschrauben des Druckstücks axial gestaucht wird, und sich dabei radial verformt, einen axialen Ansatz, welcher sich in den Kanal des Druckstücks erstreckt. Beim Festschrauben des Druckstücks wird nicht nur der Kopf des Klemmstücks gepreßt sondern dadurch, daß das Klemmstück am Anschlußstutzen befestigt ist und sich mit diesem dreht, andererseits aber auch in Reibkontakt mit der Umfangsfläche des Katheters ist, wird der Ansatz des Klemmstücks tordiert. Durch diese Torsionsverformung verringert sich wiederum der Durchmesser des Klemmstückansatzes, so daß der Katheter nicht nur im Bereich des Kopfs des Klemmstücks radial gepreßt wird sondern auch im Bereich des Klemmstückansatzes, der sich im Inneren des Kanals des Druckstücks befindet. Dadurch wird die Länge, in der der Katheter von außen geklemmt und der Katheterdurchgang abgedichtet wird, erheblich vergrößert. Ferner wird eine erhöhte Zugfestigkeit der Katheterkupplung erreicht. Außerdem erfolgt durch die Befestigung des Klemmstücks am Druckstück eine feste Zuordnung beider Teile zueinander, so daß die Katheterkupplung aus nur zwei Teilen besteht, die zusammengeschraubt werden müssen. Mindestens vor der Benutzung ist das Klemmstück Bestandteil des Druckstücks.

Die Befestigung des Klemmstücks am Druckstück kann beispielsweise durch Einspritzen, Ankleben, Klemmung oder Formschluß erfolgen. Vorteilhafterweise ist gemäß Anspruch 2 vorgesehen, daß die Haltekraft der Befestigung so gering ist, daß das Klemmstück beim Festziehen des Druckstücks von diesem abreißt. Bei der Ankopplung des Katheters, d.h. beim Einschrauben des Druckstücks in den Anschlußstutzen, setzt sich zunächst der Klemmstückkopf an der vorzugsweise konischen Druckschulter des Anschlußstutzens fest. Dabei wird der Klemmstückkopf komprimiert und gestaucht. Er kann den weiteren Drehungen des Druckstücks nicht folgen und löst sich von diesem ab. Ebenfalls löst sich der rohrförmige Ansatz des Klemmstücks vom Druckstück ab, so daß die auf den Katheter einwirkende Verwindungskraft begrenzt wird.

Mit der im Anspruch 3 angegebenen Stützkanüle wird erreicht, daß der Katheter einerseits gegen Kollabieren gesichert ist, andererseits aber auch gegen Mitdrehen mit dem Klemmstück bzw. dessen Ansatz festgehalten wird.

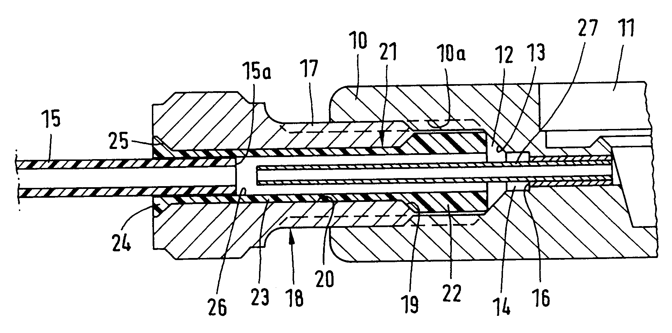

Im folgenden wird unter Bezugnahme auf die einzige Figur der Zeichnung ein Ausführungsbeispiel der Erfindung näher erläutert.

Die Zeichnung zeigt einen Längsschnitt durch die Katheterkupplung im entspannten Zustand während des Einschiebens des Katheterendes.

Die Katheterkupplung weist einen Anschlußstutzen 10 auf, der bei dem vorliegenden Ausführungsbeispiel Bestandteil eines Gehäuses 11 ist, aus dem ein flüssiges Pharmazeutikum durch den Katheter hindurch dem Körper eines Patienten zugeführt werden kann. Die Katheterkupplung kann auch an einer anderen anzuschließenden Einrichtung angebracht sein, beispielsweise an einer Spritze oder auch an einem Anschlußstück, das mit einer Schlauchleitung verbunden ist.

Im Anschlußstutzen 10 ist ein Hohlraum 12 vorhanden, der hier als Gewindebohrung ausgebildet ist, dessen Gewinde mit 10a bezeichnet ist. Die innere Begrenzung des Hohlraums 12 wird von einer schrägen Druckschulter 13 gebildet. Diese Druckschulter mündet in den zylindrischen Aufnahmeraum 14, dessen Durchmesser dem Außendurchmesser des Katheters 15 angepaßt ist und der eine Anschlagschulter 16 für das Katheterende 15a aufweist.

In das Gewinde 10a des Anschlußstutzens 10 ist das Gewinde 17 des Druckstücks 18 eingeschraubt. Am Ende des Gewindeschafts weist das Druckstück 18 eine nach Art eines Innenkonus ausgebildete Druckschulter 19 auf. Über die gesamte Länge des Druckstücks 18 erstreckt sich der Kanal 20.

Das elastomere Klemmstück 21 weist einen Kopf 22 auf, der an einem Ende an der Druckschulter 19 befestigt ist und das Druckstück 18 in axialer Richtung verlängert. Das andere Ende des Kopfes 22 stößt gegen die Druckschulter 13 des Anschlußstutzens 10. Vom Kopf 22 erstreckt sich der rohrförmige Ansatz 23 des Klemmstücks 21 durch den Kanal 20 hindurch. Am äußeren Ende ist der Ansatz 23 mit einer konischen Erweiterung 24 versehen, die in einer entsprechenden Ausnehmung 25 des Druckstücks 18 sitzt. Der axiale Kanal 26 des Klemmstücks 21 erstreckt sich mit konstantem Querschnitt über die gesamte Länge des Klemmstücks.

Am Gehäuse 11 bzw. Anschlußstutzen 10 ist eine aus starrem Material, z.B. Stahl, bestehende Stützkanüle 27 befestigt, die koaxial in den Hohlraum 12 hineinragt und mit ihrem freien Ende über das Ende des Anschlußstutzens 10 hinaus vorsteht. Der Außendurchmesser der Stützkanüle 27 entspricht etwa dem Innendurchmesser des Katheters 15, so daß der Katheter 15 ohne Überwindung größerer Reibkräfte auf die Stützkanüle 27 aufgeschoben werden kann, bis sein Ende 15a gegen die Anschlagschulter 16 stößt.

Das Klemmstück 21 ist an das Druckstück 18 angespritzt, wobei die Druckschulter 19 und die Wand des Kanals 20 Haftflächen bilden.

Die Montage der Katheterkupplung am Katheter 15 erfolgt, indem das Druckstück 18, das von dem Anschlußstutzen 10 abgeschraubt ist, auf den Katheter 15 aufgeschoben wird. Dann wird der Katheter 15 über der Stützkanüle 27 in den Anschlußstutzen 10 eingeschoben. Danach wird das Druckstück 18 zusammen mit dem an ihm befestigten Klemmstück 21 in den Hohlraum 12 eingeführt und am Anschlußstutzen 10 verschraubt. Der Kopf 22 des Klemmstücks, der im ungespannten Zustand ungehindert axial in das Innengewinde 13 eingeschoben werden kann, wird beim weiteren Festziehen des Druckstücks 18 zwischen den Druckschultern 19 und 13 gestaucht, so daß er sich radial nach außen und innen aufweitet, wobei die Reibung am Anschlußstutzen 10 immer größer wird. Dabei reißt schließlich die Haftung des Klemmstücks an der Druckschulter 19 ab. Der Ansatz 23 des Klemmstücks wird tordiert, so daß er sich zusammenschnürt und fest um die Außenfläche des Katheters 15 legt. Dabei reißt schließlich auch die Haftung des Ansatzes 23 an der Wand des Kanals 20 ab. Der Katheter 15 wird auf der Stützkanüle 27 abgestützt und durch Reibung am Mitdrehen gehindert.

Die somit hergestellte Verbindung zwischen Anschlußstutzen 10 und Katheter 15 hat eine hohe Zugfestigkeit, die im allgemeinen die Reißfestigkeit des Katheters 15 übersteigt.

Die Befestigung des Klemmstücks 21 am Druckstück 18 kann auch dadurch erfolgen, daß das vorgefertigte Klemmstück unter entsprechender Haftreibung in den Kanal 20 des Druckstücks eingesetzt ist. Druckstück und Klemmstück können also separat hergestellt und ohne Klebung oder Schweißung formschlüssig ineinandergesetzt werden.

## Patentansprüche

1. Katheterkupplung mit
   einem Anschlußstutzen (10) zum Einführen des Katheterendes,
   einem an dem Anschlußstutzen (10) verschraubten Druckstück (18), welches in den Anschlußstutzen hineinragt und eine Druckschulter (19) sowie einen Kanal (20) für den Katheter (15) aufweist, und
   einem im Anschlußstutzen (10) von dem Druckstück (18) zusammendrückbaren elastomeren Klemmstück (21),
   **dadurch gekennzeichnet**, daß das Klemmstück (21) einen an der Wand des Kanals (20) des Druckstücks (18) anliegenden rohrförmigen Ansatz (23) aufweist und an dem Druckstück (18) befestigt ist.

2. Katheterkupplung nach Anspruch 1, dadurch gekennzeichnet, daß die Befestigung des Klemmstücks (21) am Druckstück (18) eine solche Haltekraft hat, daß sie durch die Wandreibung zwischen dem mit dem Druckstück (18) gedrehten Klemmstück (21) und dem Katheter (15) bzw. dem Anschlußstutzen (10) abreißt.

3. Katheterkupplung nach Anspruch 1 oder 2, dadurch gekennzeichnet, daß am Anschlußstutzen (10) eine sich durch dessen Hohlraum (12) erstreckende Stützkanüle (27) zum Aufschieben des Katheterendes befestigt ist.

4. Katheterkupplung nach einem der Ansprüche 1 bis 3, dadurch gekennzeichnet, daß der Ansatz (23) des Klemmstücks (21) sich über die gesamte Länge des Kanals (20) des Druckstücks (18) erstreckt.

5. Katheterkupplung nach einem der Ansprüche 1 bis 4, dadurch gekennzeichnet, daß der Befestigungsbereich des Klemmstücks (21) sich mindestens teilweise über die Länge des Ansatzes (23) erstreckt.

## Claims

1. A catheter coupling comprising

a connecting member (10) for inserting the catheter end,

a thrust element (18) screwed to said connecting member (10), said thrust element extending into said connecting member and comprising a pressure receiving shoulder (19) and a channel (20) for the catheter, and

an elastomeric clamping member (21) compressed in said connecting member (10) by said thrust element (18),

**characterized in that** the clamping member (21) is provided with a tubular projection (23) that is in contact with the wall of the channel (20) of the thrust element (18) and that said clamping member is fastened to said thrust element (18).

2. The catheter coupling according to claim 1, characterized in that the fastening of the clamping member (21) to the thrust element (18) is of such a holding strength that the fastening breaks due to surface friction on the wall between the clamping member (21) rotated with the thrust element (18) and the catheter (15) or the connecting member (10), respectively.

3. The catheter coupling accroding to claim 1, characterized in that a supporting cannula (27) adapted for sliding thereon the catheter end is mounted on the connecting member (10), the supporting cannula extending through the cavity (12) in the connecting member.

4. The catheter coupling according to claim 1, characterized in that the projection (23) of the clamping member (21) extends over the whole length of the channel (20) of the thrust element (18).

5. The catheter coupling according to claim 1, characterized in that the fastening portion of the clamping member (21) extends at least partly over the length of the projection (23).

## Revendications

1. Raccord pour cathéter comportant

un embout de raccordement (10) pour insérer l'extrémité du cathéter,

une pièce de pression (18) qui est vissée sur l'embout de raccordement (10), qui pénètre dans l'embout de raccordement et qui comporte un épaulement de pression (19) ainsi qu'un canal (20) pour le cathéter (15), et

une pièce de serrage (21) en élastomère, qui, dans l'embout de raccordement (10), peut être comprimée par la pièce de pression (18),

caractérisé en ce que la pièce de serrage (21) comporte un appendice (23) de forme tubulaire s'appuyant contre la paroi du canal (20) de la pièce de pression (18), et est fixée à la pièce de pression (18).

2. Raccord pour cathéter selon la revendication 1, caractérisé en ce que la fixation de la pièce de serrage (21) sur la pièce de pression (18) présente une force de maintien telle, qu'elle est interrompue par arrachement en raison du frottement de paroi entre la pièce de serrage (21) qui tourne avec la pièce de pression (18), et le cathéter (15) ainsi que l'embout de raccordement (10).

3. Raccord pour cathéter selon la revendication 1 ou 2, caractérisé en ce que sur l'embout de raccordement (10) est fixé une canule de soutien (27) qui s'étend au travers de la cavité (12) de l'embout de raccordement et qui est destinée à l'emmanchement de l'extrémité du cathéter.

4. Raccord pour cathéter selon l'une des revendications 1 à 3, caractérisé en ce que l'appendice (23) de la pièce de serrage (21) s'étend sur la totalité de la longueur du canal (20) de la pièce de pression (18).

5. Raccord pour cathéter selon l'une des revendications 1 à 4, caractérisé en ce que la zone de fixation de la pièce de serrage (21) s'étend sur au moins une partie de la longueur de l'appendice (23).